# EUROPÄISCHE PATENTANMELDUNG

(11) **EP 4 403 198 A1**
(43) Veröffentlichungstag der Anmeldung: **24.07.2024**
(21) Anmeldenummer: 23152207.9
(22) Anmeldetag: 18.01.2023
(51) Int. Cl.: A61M 5/14, A61M 39/08

(54) **HALTERUNG FÜR MEDIZINISCHE ANSCHLUSSLEITUNGEN**

(71) Anmelder: Wintjens, Peter, 47533 Kleve (DE); Wintjens, Felix, 47533 Kleve (DE)
(72) Erfinder: Wintjens, Peter, 47533 Kleve (DE); Wintjens, Felix, 47533 Kleve (DE)
(74) Vertreter: Seyer & Nobbe Patentanwälte PartmbB

(57) **Zusammenfassung**

Die Erfindung betrifft eine Halterung 1 für medizinisch notwendige Anschlussleitungen, insbesondere für Infusions- und Perfusionsleitungen sowie Kabelverbindungen oder Verbindungsleitungen für operative Instrumente, bestehend zumindest aus einem Grundkörper 2, welcher arretierbar ist und mindestens eine Aufnahmeöffnung 10 für eine Anschlussleitung aufweist. Um die medizinisch notwendigen Anschlussleitungen übersichtlich anzuordnen besitzt der Grundkörper 2 eine Ausnehmung 8, welche zum Verbinden mit medizinischen Geräten oder Einrichtungen vorgesehen ist und zumindest eine, vorzugsweise eine Vielzahl von Aufnahmeöffnungen 10 mit einem reduzierten Öffnungsbereich 15, um beispielsweise Anschlussleitungen einklemmend aufnehmen zu können.

## Beschreibung

Die Erfindung betrifft eine Halterung für medizinisch notwendige Anschlussleitungen, insbesondere für Infusions- und Perfusionsleitungen sowie Kabelverbindungen oder Verbindungsleitungen für operative Instrumente, bestehend zumindest aus einem Grundkörper, welcher arretierbar ist und mindestens eine Aufnahmeöffnung für eine Anschlussleitung aufweist.

In Krankenhäusern und Pflegeheimen werden zur Versorgung der Patienten, insbesondere bei einer Intensivpflege mehrere Geräte benötigt, um die Parameter des menschlichen Körpers zu erfassen. Über Zugänge zum menschlichen Körper werden zu diagnostischen oder therapeutischen Zwecken Stoffe oder Instrumente zum oder in den Körper geleitet. Zeitgleich werden Stoffe oder Signale über die Zugänge vom Körper erfasst, um diese auszuwerten. Die Anschlussleitungen versorgen den menschlichen Körper mit Stoffdepots, beispielsweise Infusionsbeutel oder Infusionsspritzen. Über Endgeräte, die die Zustände beziehungsweise Körperfunktionen messen und grafisch aufbereiten erfolgt eine Überwachung mit notwendiger Signalfunktion. Aus dem Körper entnommene Stoffe können gegebenenfalls verändert und wieder zurückgeführt werden. Beispielsweise werden in der operativen Anästhesie und Intensivmedizin unter anderem zentral-venöse Katheter, Pulmonalikatheter, arterielle Druckmessungen und periphere venöse Zugänge zu den verwendeten Zugangswegen für das hämodynamische Monitoring der Patienten und als Zugangswege für Infusionen und Medikamente benötigt.

Sämtliche verwendeten Anschlussleitungen befinden sich in unmittelbarer Nähe des Patienten und überlagern sich räumlich sehr oft, sodass es zu einem räumlichen Wirrwarr kommen kann und erst nach längerem Suchen einzelne Leitungen auffindbar sind. Hierzu bedarf es teilweise einer Auftrennung vorhandener Leitungen, welche neu sortiert werden müssen und können in Akutsituationen oder bei hämodynamisch instabilen Patienten zu Problemen führen. Die Probleme entstehen einerseits bei den pflegerischen Mitarbeitern, aber ebenso können diese zu Komplikationen bei den Patienten führen. Gerade bei Intensivpatienten besteht zudem die Notwendigkeit diese in regelmäßigen Abständen umzulagern, sodass im Laufe der Zeit eine nicht mehr überschaubare Unordnung an Zuleitungen entsteht.

Durch das Fehlen geeigneter Halterungen liegen oder hängen die Anschlussleitungen auf den Boden, wodurch eine Keimstraße und damit potentielle Infektionen entstehen können. Hierbei handelt es sich teilweise um invasive Zu-/Abgänge, die nicht in Bodenkontakt stehen dürfen. Die Anschlussleitungen unterliegen hierbei der Schwerkraft und werden beim Verstellen des Bettes in ihrer Lage verändert und können sich somit verhaken, verwickeln, eingeklemmt werden und sind möglicherweise Zugbelastungen ausgesetzt. Diese wesentlichen Nachteile können sogar zu einer Patientengefährdung führen. Darüber hinaus passiert es regelmäßig, dass die Personen in den Zu-/Abgängen, die auf dem Boden liegen, verfangen, sodass es zu Systemschäden und gezogenen Zu-/Abgängen führt.

Der vorliegenden Patentanmeldung liegt daher die Aufgabenstellung zugrunde, eine Halterung für medizinisch notwendige Anschlussleitungen aufzuzeigen, welche die Möglichkeit bietet, eine Ordnung herzustellen, um einen besseren Überblick zu erlangen und im Bedarfsfall gezielt einzelne Leitungen zu entfernen oder zu erneuern.

Erfindungsgemäß ist zur Lösung der Aufgabenstellung vorgesehen, dass der Grundkörper eine Ausnehmung aufweist, welche zur Verbindung mit medizinischen Geräte oder Einrichtungen vorgesehen ist und die wenigstens eine Aufnahmeöffnung einen im Querschnitt reduzierten Öffnungsbereich aufweist. Weitere vorteilhafte Ausgestaltungen der Erfindung ergeben sich aus den Unteransprüchen.

Die Erfindung schlägt eine Halterung vor, die aus einem Grundkörper besteht, welcher eine erste Ausnehmung aufweist. Diese Ausnehmung ist dazu gedacht den Grundkörper an vorhandene medizinischen Geräten oder Einrichtungen zu befestigen, vorzugsweise aufzuklemmen. Die vorhandene Ausnehmung ermöglicht hierbei die Halterung in den unterschiedlichsten, zweckdienlichen Positionen anzubringen. Durch eine klemmende Aufnahme der Ausnehmung kann hierbei die Position der Halterung jederzeit wieder verändert werden, wobei vorzugsweise ein Aufklemmen beispielsweise auf die Umrandung eines Krankenbettes oder weiteren medizinischen Einrichtung erfolgen kann, die zur Überwachung des Patienten notwendig sind.

Damit die Anschlussleitungen geordnet und übersichtlich mithilfe der Halterung angeordnet werden können, weist diese wenigstens eine Aufnahmeöffnung, vorzugsweise eine Vielzahl von Aufnahmeöffnungen auf. Die einzelnen Aufnahmeöffnungen weisen im Querschnitt betrachtet hierbei einen reduzierten Öffnungsbereich auf, sodass die Anschlussleitungen durch den Öffnungsbereich hindurchgeführt, vorzugsweise eingedrückt werden und in einen vorhandenen größeren Durchgangsbereich einliegen. Auf diese Weise wird sichergestellt, dass die Anschlussleitungen bei einer Umlagerung der Patienten im Bett oder Neuordnung der medizinischen Geräte durch die jeweiligen Aufnahmeöffnungen verschieblich nachgeführt werden können. Darüber hinaus besteht bei einem notwendigen Austausch einer Anschlussleitung ohne weiteres die Möglichkeit diese zu entfernen und gegebenenfalls zu ersetzen.

Die erfindungsgemäße Halterung ermöglicht eine kontrollierte Führung der Anschlussleitungen ohne die Hygienerichtlinien zu verletzen und den Patienten zu gefährden. Dies wird insbesondere dadurch gewährleistet, dass ein Bodenkontakt ausgeschlossen wird. Darüber hinaus verhindert die Halterung, dass Verhaken, Verwickeln, Einklemmen von Anschlussleitungen und der darauf möglicherweise entstehenden Zugbelastungen.

Um die Halterung und den Grundkörper mit den unterschiedlichsten Pflegebetten oder medizinischen Einrichtungen verbinden zu können ist in Ausgestaltung der Erfindung vorgesehen, dass der Grundkörper eine rechteckige, runde oder eine U-förmige aufweist. Die Formgebung des Grundkörpers kann hierbei so gewählt werden, dass diese sich an die Form der medizinischen Geräte oder Einrichtungen anpasst und saubere Übergänge entstehen. Diese Übergange sind auch notwendig, damit beispielsweise die Reinigung der Pflegebetten oder medizinischen Einrichtungen erleichtert wird und die Ablagerung von Schmutz unterhalb der Halterung vermieden wird.

In weiterer Ausgestaltung der Erfindung ist vorgesehen, dass der Grundkörper eine rechteckige, runde, ovale oder U-förmige Ausnehmung aufweist. Vorzugsweise werden gerade bei Pflegebetten oder Krankenhausbetten runde Gestänge verwendet, sodass die Halterung bündig auf die verschiedenen Ausführungen von Gestängen aufgeklemmt werden kann. Es besteht aber ebenso die Möglichkeit, dass die oberen Abschlussleisten eines Pflegebettes eine rechteckförmige Form aufweisen, welche beispielsweise im Kantenbereich abgerundet ist, sodass auch für derartige Ausführungsvarianten die Möglichkeit besteht die Halterung aufzuklemmen. Somit kann die Halterung flächenbündig auf die unterschiedlichsten Formen der Gestänge von Pflegebetten oder medizinischen Einrichtungen aufgeklemmt werden, ohne dass hervorstehende Ecken oder Überstände zu einer Verletzungsgefahr führen, aber ebenso wird auch die Reinigung der Halterung wesentlich erleichtert. Hierbei bietet es sich vorzugsweise an, dass der Grundkörper entsprechend der Form der medizinischen Geräte oder Einrichtungen angepasst ist und eine entsprechende Ausnehmung aufweist, um eine sichere Aufklemmung zu gewährleisten.

Beispielsweise besteht die Möglichkeit, dass der Grundkörper in U-Form zwei gekrümmte Schenkel aufweist, welche ein rundes Gestänge der Krankenhausbetten oder Pflegebetten umschließt und damit einen besonders sicheren Halt gewährleistet. In ähnlicher Weise können auch abgerundete rechteckförmige Abschlussleisten der Pflegebetten umklammert werden. Zu diesem Zweck ist zumindest ein Schenkel, vorzugsweise beide Schenkel elastisch ausgebildet, damit die beiden Schenkel aufgebogen werden können und nach dem Aufklemmen wieder die ursprüngliche Form einnehmen.

In weiterer Ausgestaltung der Erfindung ist vorgesehen, dass der Grundkörper auf der diametral der Ausnehmung abgewandten Seite einen Steg mit mehreren Aufnahmeöffnungen aufweist. Diese Aufnahmeöffnungen sind zur Aufnahme der Anschlussleitungen vorgesehen und befinden sich somit im oberen Bereich der Halterung und sind damit leicht zugänglich. Die Anschlussleitungen können somit nach der Verlegung unmittelbar in die Aufnahmeöffnungen eingedrückt werden und können im Bedarfsfall jederzeit umgruppiert werden. Hierbei ist der Grundkörper und der Steg vorzugsweise einstückig ausgebildet, sodass eine besonders kostengünstige Herstellung möglich ist. Selbstverständlich besteht alternativ die Möglichkeit den Grundkörper und den Steg jeweils einzeln zu fertigen und mit entsprechenden Verbindungsmitteln zu verbinden.

In weiterer Ausgestaltung der Erfindung ist vorgesehen, dass die Aufnahmeöffnungen teilweise unterschiedlich große Abmessungen aufweisen, und/oder dass die Aufnahmeöffnungen zumindest teilweise in äquidistanten Abständen angeordnet sind, um auf diese Weise eine optische Trennung von fluidführenden, gasführenden Anschlussleitungen und elektrischen Leitungen herbeizuführen. Mithilfe der Halterung besteht somit die Möglichkeit, die verschiedenen unterschiedlichen Anschlussleitungen, sei es zum Fluidtransport, Gastransport oder elektrischen Versorgungsleitungen voneinander zu trennen und zu gruppieren ohne den Überblick zu verlieren. Die unterschiedlich großen Abmessungen der Aufnahmeöffnungen ermöglichen eine entsprechende Auswahl der verwendeten Aufnahmeöffnung vorzunehmen, wenn teilweise dünnere oder dickere Anschlussleitungen aufgenommen werden sollen. Zur weiteren Trennung ist vorgesehen, dass die unterschiedlich großen Aufnahmeöffnungen gruppenweise zusammengefasst sind, sodass sich auf der oberen Seite der Halterung eine übersichtlich Anordnung der einzelnen Anschlussleitungen ergibt und damit die Übersichtlichkeit verbessert wird.

In Weiterer Ausgestaltung ist vorgesehen, dass der Steg zumindest im Bereich der Aufnahmeöffnungen elastisch zurückfedernd ausgebildet ist. Hierdurch besteht die Möglichkeit, dass die Anschlussleitungen durch den Öffnungsbereich hindurch ebenfalls aufgeklemmt werden und wieder entfernbar sind soweit dies bei dem Wegfall des einen oder anderen medizinischen Gerätes notwendig ist.

Zur Erhöhung der Klemmsicherheit ist im Weiteren vorgesehen, dass der Öffnungsbereich der Aufnahmeöffnungen durch Verdickungen der auslaufenden Schenkel verringert ist, sodass ein Herausfallen einzelner Anschlussleitungen nicht ohne einen manuellen Eingriff möglich ist. Zur erleichterten Zuführung der Anschlussleitungen ist im Weiteren vorgesehen, dass die Verdickungen zur Außenseite hin mit gegenüberliegenden Abschrägungen versehen sind, um die Anschlussleitungen in den Öffnungsbereich ohne Beschädigung einklemmend eindrücken zu können.

Die vorliegende Erfindung zeichnet sich dadurch aus, dass mithilfe der Halterung eine für sämtliche beteiligten Personen leicht erkennbare Zuordnung der Anschlussleitungen möglich ist, weil diese in der Halterung in den vorhandenen Aufnahmeöffnungen positioniert werden können und jederzeit ein Austausch oder ein Versatz möglich ist. Die Anschlussleitungen werden hierbei in einer Aufnahmeöffnung gehalten, die einen verengten Öffnungsbereich aufweist, der gleichzeitig verhindert, dass einzelne Anschlussleitungen wieder aus den Ausnahmeöffnungen herausgleiten können und nur bei einem manuellen Eingriff somit entfernbar sind. Mithilfe der Halterung besteht somit die Möglichkeit die verschiedenen unterschiedlichen Anschlussleitungen voneinander zu trennen und zu gruppieren ohne den Überblick zu verlieren. Die Halterung ist somit für die Ärzte und Pflegekräfte äußerst vorteilhaft, weil die Übersichtlichkeit verbessert wird und eine Leitungsführung durch die Halterung eine saubere und nachvollziehbare Leitungsanordnung möglich ist.

Die Erfindung wird im Weiteren anhand der Figuren näher erläutert.

Es zeigt
- Fig. 1: in einer perspektivischen Ansicht eine Halterung für medizinisch notwendige Anschlussleitungen und
- Fig. 2: in drei weiteren Ansichten die Halterung gemäß Figur 1

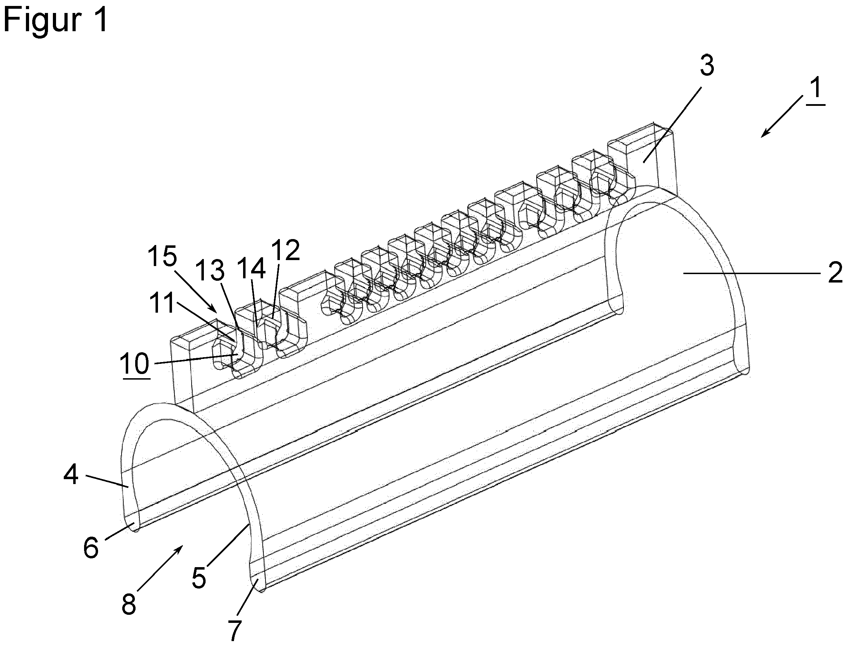

Figur 1 zeigt eine Halterung 1, bestehend aus einem Grundkörper 2 und einem Steg 3, welche einstückig hergestellt werden können oder gegebenenfalls nach separater Herstellung miteinander verbunden werden können. Der Grundkörper 2 ist im gezeigten Ausführungsbeispiel annähernd halbrund ausgeführt und besitzt zwei gekrümmte Schenkel 4, 5 mit jeweils einer Verdickung 6, 7 an den Schenkelenden. Die Form der beiden Schenkel 4, 5 wurde so gewählt, dass die Halterung 1 beispielsweise auf ein rundes Gestänge des Pflegebettes oder sonstige medizinische Einrichtungen aufgeklemmt werden kann. Der Abstand der beiden Schenkel 4, 5 ist bei der Ausnehmung 8 im Querschnitt verringert, sodass nach dem Aufklemmen ein sicherer Halt gewährleistet wird. Zu dem sicheren Halten gehen auch die beiden Verdickungen 6, 7, sodass die Halterung 1 nicht unabsichtlich abrutschen kann, sondern nur durch manuelle Kraftaufwendung wieder entfernbar ist.

Der Steg 3 ist mit einer Vielzahl von Aufnahmeöffnungen 10 ausgestattet, die zum Teil im Querschnitt betrachtet einen unterschiedlichen Durchmesser oder Durchbruch aufweisen, wobei die auslaufenden Schenkel 11, 12 ebenfalls eine Verdickung 13, 14 aufweisen, um ein Herausrutschen der aufzunehmenden Anschlussleitungen zu verhindern. Der Öffnungsbereich 15 der jeweiligen Aufnahmeöffnungen 10 ist hierbei so gewählt worden, dass die Anschlussleitungen durch den verengten Öffnungsbereich 15 hindurchgedrückt werden können und sicher in dem tieferliegenden runden oder eckigen Querschnitt im gezeigten Ausführungsbeispiel zu liegen kommen können. Die Aufnahmeöffnungen 10 sind mit unterschiedlichen Querschnitten ausgestattet, wobei zusätzlich eine Gruppierung der unterschiedlichen Querschnitte vorliegt, sodass Anschlussleitungen mit unterschiedlichen Durchmessern jeweils in die zugehörige Aufnahmeöffnung eingeklemmt werden können. Anschlussleitungen, wie sie hier verwendet werden können, sind zeichnerisch nicht dargestellt.

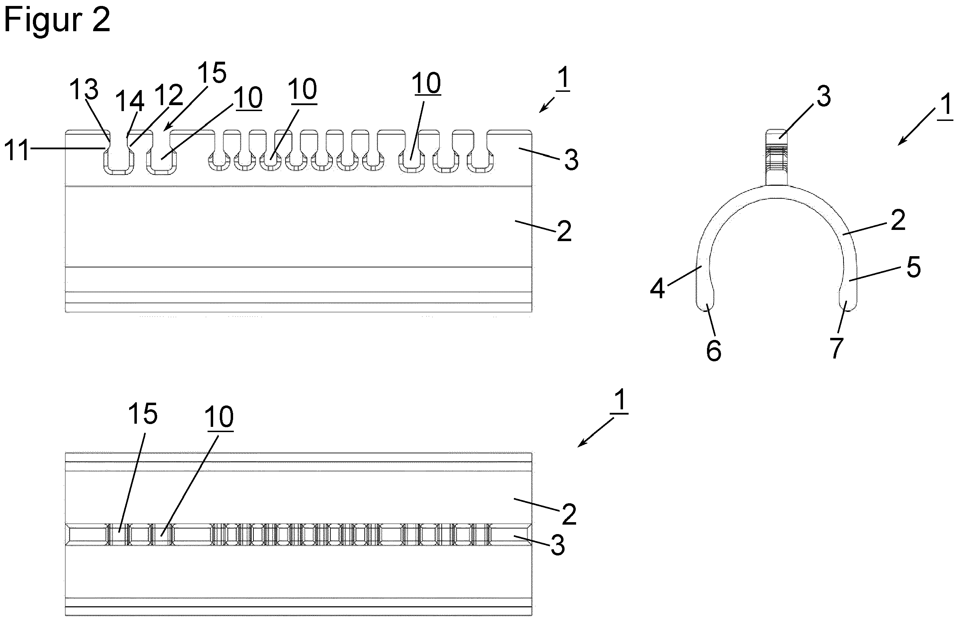

Figur 2 zeigt in drei Ansichten ebenfalls die Halterung 1. Aus einer Seitenansicht und Draufsicht ist insbesondere der Grundkörper 2 sowie der Steg 3 erkennbar, wobei die einzelnen Aufnahmeöffnungen 10 eine unterschiedliche Größe aufweisen und jeweils auslaufende Schenkel 11, 12 mit einer Verdickung 13 versehen sind, sodass die Anschlussleitungen durch den Öffnungsbereich 15 hindurch einklemmend eingedrückt werden können. Die einzelnen Aufnahmeöffnungen 10 sind entsprechend ihren Öffnungsquerschnitten gruppenweise angeordnet, sodass beispielsweise elektrische Anschlussleitungen oder auch fluidführende Anschlussleitungen getrennt angeordnet werden können. Aus der Draufsicht ist ersichtlich, dass der Steg 3 über die gesamte Länge des Grundkörpers 2 ausgebildet ist. Aus einer stirnseitigen Ansicht ist nochmals der Grundkörper 2 mit den zur Einklemmung vorgesehenen Schenkel 4, 5 und den endseitigen Verdickungen 6, 7 ersichtlich, wobei die beiden Schenkel 4, 5 nahezu kreisförmig über einen Winkelbereich von mehr als 180 Grad ausgebildet ist, sodass eine rohrförmige Verbindungsstrebe eines Pflegebettes oder dergleichen einklemmend aufgenommen werden kann.

### Bezugszeichenliste

- 1: Halterung
- 2: Grundkörper
- 3: Steg
- 4: Schenkel
- 5: Schenkel
- 6: Verdickung
- 7: Verdickung
- 8: Ausnehmung
- 10: Aufnahmeöffnung
- 11: Schenkel
- 12: Schenkel
- 13: Verdickung
- 14: Verdickung
- 15: Öffnungsbereich

## Patentansprüche

1. Halterung (1) für medizinisch notwendige Anschlussleitungen, insbesondere für Infusions- und Perfusionsleitungen sowie Kabelverbindungen oder Verbindungsleitungen für operative Instrumente, bestehend zumindest aus einem Grundkörper (2), welcher arretierbar ist und mindestens eine Aufnahmeöffnung (10) für eine Anschlussleitung aufweist,
**dadurch gekennzeichnet,**
**dass** der Grundkörper (2) eine Ausnehmung (8) aufweist, welche zur Verbindung mit medizinischen Geräte oder Einrichtungen vorgesehen ist und die wenigstens eine Aufnahmeöffnung (10) einen im Querschnitt reduzierten Öffnungsbereich (15) aufweist.

2. Halterung (1) nach Anspruch 1,
**dadurch gekennzeichnet,**
**dass** der Grundkörper (2) eine rechteckige, runde oder eine U-förmige Form aufweist.

3. Halterung (1) nach Anspruch 1 oder 2,
**dadurch gekennzeichnet,**
**dass** der Grundkörper (2) eine rechteckige, runde, ovale oder U-förmige Ausnehmung (8) aufweist.

4. Halterung (1) nach einem der Ansprüche 1, 2 oder 3,
**dadurch gekennzeichnet,**
**dass** der Grundkörper (2) in U-Form zwei gekrümmte Schenkel (4, 5) aufweist.

5. Halterung (1) nach einem der Ansprüche 1 bis 4,
**dadurch gekennzeichnet,**
**dass** zumindest ein Schenkel (4, 5) elastisch ausgebildet ist.

6. Halterung (1) nach einem der Ansprüche 1 bis 5,
**dadurch gekennzeichnet,**
**dass** der Grundkörper (2) auf der diametral der Ausnehmung (8) abgewandten Seite einen Steg (3) mit mehreren Aufnahmeöffnungen (10) aufweist.

7. Halterung (1) nach einem der Ansprüche 1 bis 6,
**dadurch gekennzeichnet,**
**dass** der Grundkörper (2) und der Steg (3) einstückig ausgebildet sind.

8. Halterung (1) nach einem der Ansprüche 1 bis 7,
**dadurch gekennzeichnet,**
**dass** die Aufnahmeöffnungen (10) teilweise unterschiedlich große Abmessungen aufweisen, und/oder dass die Aufnahmeöffnungen (10) zumindest teilweise in äquidistanten Abständen angeordnet sind.

9. Halterung (1) nach einem der Ansprüche 1 bis 8,
**dadurch gekennzeichnet,**
**dass** die unterschiedlich großen Aufnahmeöffnungen (10) gruppenweise zusammengefasst sind.

10. Halterung (1) nach einem der Ansprüche 1 bis 9,
**dadurch gekennzeichnet,**
**dass** der Steg (3) zumindest im Öffnungsbereich (15) der Aufnahmeöffnungen (10) teilweise elastisch zurückfedern ausgebildet ist.

11. Halterung (1) nach einem der Ansprüche 1 bis 10,
**dadurch gekennzeichnet,**
**dass** der Öffnungsbereich (15) der Aufnahmeöffnungen (10) durch Verdickungen (6, 7, 12, 13) der auslaufenden Schenkel (11, 12) verkleinert ist.

12. Halterung (1) nach einem oder mehreren der Ansprüche 1 bis 11,
**dadurch gekennzeichnet,**
**dass** die Verdickungen (6, 7, 12, 13) zur Außenseite hin mit gegenüberliegenden Abschrägungen versehen sind.

## Geänderte Patentansprüche

### Geänderte Patentansprüche gemäss Regel 137(2) EPÜ.

1. Halterung (1) für medizinisch notwendige Anschlussleitungen, insbesondere für Infusions- und Perfusionsleitungen sowie Kabelverbindungen oder Verbindungsleitungen für operative Instrumente, bestehend zumindest aus einem Grundkörper (2), welcher arretierbar ist und eine Ausnehmung (8) aufweist, welche zur Verbindung mit medizinischen Geräten oder Einrichtungen vorgesehen ist und mindestens eine Aufnahmeöffnung (10) für eine Anschlussleitung aufweist, welche einen im Querschnitt reduzierten Öffnungsbereich (15) aufweist,
**dadurch gekennzeichnet,**
**dass** der Grundkörper (2) eine rechteckige, runde, ovale oder U-förmige Ausnehmung (8) aufweist, welche durch zwei Schenkel (4, 5) begrenzt ist, an deren Enden Verdickungen (6, 7) vorhanden sind.

2. Halterung (1) nach Anspruch 1,
**dadurch gekennzeichnet,**
**dass** der Grundkörper (2) eine rechteckige, runde oder eine U-förmige Form aufweist.

3. Halterung (1) nach Anspruch 1 oder 2,
**dadurch gekennzeichnet,**
**dass** der Grundkörper (2) in U-Form zwei gekrümmte Schenkel (4, 5) aufweist.

4. Halterung (1) nach einem der Ansprüche 1, 2 oder 3,
**dadurch gekennzeichnet,**
**dass** zumindest ein Schenkel (4, 5) elastisch ausgebildet ist.

5. Halterung (1) nach einem der Ansprüche 1 bis 4,
**dadurch gekennzeichnet,**
**dass** der Grundkörper (2) auf der diametral der Ausnehmung (8) abgewandten Seite einen Steg (3) mit mehreren Aufnahmeöffnungen (10) aufweist.

6. Halterung (1) nach einem der Ansprüche 1 bis 5,
**dadurch gekennzeichnet,**
**dass** der Grundkörper (2) und der Steg (3) einstückig ausgebildet sind.

7. Halterung (1) nach einem der Ansprüche 1 bis 6,
**dadurch gekennzeichnet,**
**dass** die Aufnahmeöffnungen (10) teilweise unterschiedlich große Abmessungen aufweisen, und/oder dass die Aufnahmeöffnungen (10) zumindest teilweise in äquidistanten Abständen angeordnet sind.

8. Halterung (1) nach einem der Ansprüche 1 bis 7,
**dadurch gekennzeichnet,**
**dass** die unterschiedlich großen Aufnahmeöffnungen (10) gruppenweise zusammengefasst sind.

9. Halterung (1) nach einem der Ansprüche 1 bis 8,
**dadurch gekennzeichnet,**
**dass** der Steg (3) zumindest im Öffnungsbereich (15) der Aufnahmeöffnungen (10) teilweise elastisch zurückfedern ausgebildet ist.

10. Halterung (1) nach einem der Ansprüche 1 bis 9,
**dadurch gekennzeichnet,**
**dass** der Öffnungsbereich (15) der Aufnahmeöffnungen (10) durch Verdickungen (12, 13) der auslaufenden Schenkel (11, 12) verkleinert ist.

11. Halterung (1) nach einem der Ansprüche 1 bis 10,
**dadurch gekennzeichnet,**
**dass** die Verdickungen (12, 13) zur Außenseite hin mit gegenüberliegenden Abschrägungen versehen sind.
